# EUROPÄISCHE PATENTSCHRIFT

(11) **EP 1 945 239 B1**
(45) Veröffentlichungstag und Bekanntmachung des Hinweises auf die Patenterteilung: **18.05.2016**
(21) Anmeldenummer: 06805489.9
(22) Anmeldetag: 26.10.2006
(51) Int. Cl.: A61K 36/88

(54) **ZUBEREITUNG UND ANWENDUNG EINES PHYTOTHERAPEUTIKUMS**
PREPARATION AND USE OF A PHYTOTHERAPEUTIC AGENT
PREPARATION ET UTILISATION D'UN AGENT PHYTOTHERAPEUTIQUE

(30) Priorität: 27.10.2005 DE 102005051631; 08.11.2005 US 734283 P
(43) Veröffentlichungstag der Anmeldung: 23.07.2008
(73) Patentinhaber: SUSILO, Gisela, 50999 Köln (DE)
(72) Erfinder: SUSILO, Gisela, 50999 Köln (DE)
(74) Vertreter: Arth, Hans-Lothar
(86) Internationale Anmeldenummer: PCT/DE2006/001892
(87) Internationale Veröffentlichungsnummer: WO 2007/048398

(56) Entgegenhaltungen:
- SOUTHWELL K ET AL: "CHEMICAL CHARACTERISTICS OF PANDANUS-CONOIDEUS FRUIT LIPID" JOURNAL OF THE SCIENCE OF FOOD AND AGRICULTURE, Bd. 58, Nr. 4, 1992, Seiten 593-594, XP002431636 ISSN: 0022-5142
- PEUNGVICHA PENCHOM ET AL: "4-Hydroxybenzoic acid: A hypoglycemic constituent of aqueous extract of Pandanus odorus root" JOURNAL OF ETHNOPHARMACOLOGY, Bd. 62, Nr. 1, August 1998 (1998-08), Seiten 79-84, XP002431637 ISSN: 0378-8741
- ROUT ET AL: "Extraction of Kewda [Pandanus fascicularis] Lam. flowers with hexane: composition of concrete, absolute and wax" MEDICINAL & AROMATIC PLANTS ABSTRACTS, NATIONAL INSTITUTE OF SCIENCE COMMUNICATION AND INFORMATION, IN, Bd. 27, Nr. 5, Oktober 2005 (2005-10), XP018005160 ISSN: 0250-4367
- ABDUL MUN'IM ET AL.: "UJI HAMBATAN TUMORIGENESIS SARI. BUAH MERAH (PANDANUS CONOIDEUS. LAM.) TERHADAP TIKUS PUTIH BETINA. YANG DIINDUKSI 7,12. DIMETILBENZ(a)ANTRASEN (DMBA)" MAJALAH ILMU KEFARMASIAN, Bd. 3, Nr. 3, Dezember 2006 (2006-12), Seiten 153-161, XP002431638
- I Made Budi, Fendy R. Paimin: "Buah Merah", June 2005 (2005-06), Penebar Swadaya, Jakarta ISBN: 979-489-883-X pages 1-75,
- H. Machud Yahya, Bernard T. Wahyu Wiryanta: "Khasiat dan manfaat buah mehra si emas merah dari papua", February 2005 (2005-02), PT AgroMedia Pustaka, Jakarta ISBN: 979-3702-30-3 pages 1-84,

## Beschreibung

Die Erfindung betrifft biologisch aktive lipophile Extrakte (Öl-Fraktion) sowie hydrophile Extrakte aus der Frucht der Pflanze Pandanus conoideus sowie das schonende Verfahren zur Herstellung dieser biologisch aktiven Extrakte, insbesondere die Kaltpressung zur Ölgewinnung. Ferner wird die Verwendung dieser Extrakte zur Herstellung einer pharmazeutischen Zusammensetzung sowie deren Anwendungsgebiete beispielsweise in der Krebstherapie beschrieben.

Pandanus conoideus ist eine endemische Pflanzenart aus Irian Jaya, Indonesien. Die Früchte dieser Pflanze finden Anwendung bei den Einheimischen als Nahrungsmittel, Nahrungsmittelergänzung, Viehfutter, natürlicher Farbstoff und als Heilmittel gegen verschiedenste Erkrankungen wie Würmererkrankungen, Blindheit, Hauterkrankungen sowie als Mittel zur Wundbehandlung.

Die traditionelle Zubereitung erfolgt durch langes Kochen der roten äußeren Fruchtschicht und anschließendem Zerreiben des Fruchtfleisches, um einen kernfreien Brei zu erhalten. Der daraus gewonnene ölige Brei wird als tägliches Lebensmittel oder als Heilmittel eingesetzt.

Aufgabe der vorliegenden Erfindung ist es, die pharmakologisch aktiven Inhaltsstoffe der Frucht des Pandanus conoideus in aufkonzentrierter Form bereitzustellen sowie weitere Anwendungsfelder für die Wirkstoffextrakte gewonnen aus der Frucht des Pandanus conoideus zu erschließen.

Diese Aufgabe wird durch die technische Lehre der unabhängigen Patentansprüche gelöst. Vorteilhafte Ausgestaltungen der Erfindung sind in den abhängigen Patentansprüchen, der Beschreibung sowie den Beispielen angegeben.

Überraschenderweise wurde gefunden, dass 1) ein durch die direkte Verpressung des roten Fruchtfleisches gewonnenes Öls, 2) das durch eine Extraktion des roten Fruchtfleisches mit organischen Lösungsmitteln unterhalb von 40°C gewonnenes Öls, 3) die alkoholischen Extrakte des roten Fruchtfleisches, 4) die alkoholischen Extrakte der Kerne, 5) der durch die direkte Verpressung des hellen inneren Fruchtanteils gewonnene, wässrige Saft und 6) die hydrophilen/alkoholischen Extrakte des getrockneten hellen, inneren Fruchtanteil aus der Frucht der Pflanze Pandanus conoideus einzeln und zusammen wirksam gegen eine Vielzahl von Krebserkrankungen sind wie beispielsweise Bauchspeichetdrüsenkrebs. Blasenkrebs, Lungenkrebs und Brustkrebs.

Somit betrifft die vorliegende Erfindung ein Verfahren zur Herstellung eines lipophilen Extraktes aus dem roten Fruchtfleisch der Früchte der Spezies Pandanus conoideus durch die direkte Verpressung, d.h. Kaltpressung des abgetrennten roten Fruchtfleisches bevorzugt ohne jegliche chemische Zusätze bei Temperaturen vorzugsweise unterhalb von 40°C oder durch Extraktion des abgetrennten zerkleinerten roten Fruchtfleisches mittels organischer Lösungsmittel bei Temperaturen unterhalb von 40°C. Insbesondere bevorzugt ist jedoch die Kaltpressung unterhalb von 40°C da zum einen festgestellt worden ist, dass beim Kochen des roten Fruchtfleisches in Wasser oder einem organischen Lösungsmittel wichtige bioaktive Stoffe wie beispielsweise Tocopherole und andere Vitamine, Phytosterole etc. zerstört werden. Darüber hinaus wird ein durch Kochen hergestellter Extrakt sehr schnell ranzig. Durch die erfindungsgemäße Kaltpressung erhält man überraschenderweise Extrakte, d.h. Öle, welche mindestens ein Jahr und wahrscheinlich noch deutlich länger stabil sind. Die bisherigen Stabilitätsdaten zeigen eine Stabilität von einem Jahr von Proben, welche bisher ein Jahr gelagert worden sind. Die Stabilitätsprüfung wird weiter fortgesetzt und es werden noch deutlich längere Stabilitäten erwartet.

Eine Temperaturerhöhung während des Extraktionsverfährens oder des Verarbeitungsverfahrens des roten Fruchtfleisches über 40°C führt überraschenderweise zu einer Verringerung der Stabilität und Lagerstabilität des erhaltenen Extraktes. Gleiches gilt bei dem Zusatz organischer Lösungsmittel während der Extraktion. Je länger die Temperatur während der Extraktion des roten Fruchtfleisches über 40°C liegt und je höher die Temperatur während des Herstellungsprozesses des Öles aus dem rotem Fruchtfleisch über 40°C liegt, desto niedriger wird die Stabilität des Extraktes. Somit ist es wichtig, bei dem gesamten Herstellungsverfahrens des Öles aus dem roten Fruchtfleisch darauf zu achten, dass die Extraktions- und Verarbeitungsbedingungen eine Temperatur von 40°C nicht überschreiten und im Idealfall der gesamte Prozess der Extraktion und Ölgewinnung bei Raumtemperatur durchgeführt wird.

Bekannte Verfahren zur Extraktion des roten Fruchtfleisches umfassen das Kochen des roten Fruchtfleisches eventuell mit Zusatz von Wasser, Herstellung eines Breis und Abschöpfen des auf dem Brei schwimmenden Öls und führen zu Ölen, welche schnell ranzig werden. Dieses Öl wird von den Einheimischen gegen beliebige Beschwerden eingesetzt, wobei eine wissenschaftliche Grundlage für die Wirksamkeit des Öles fehlt.

So beschreiben I Made Budi, FendyR. Paimin (in "Buah Merah", Juni 2005, Penebar Swadaya Jakarta, Seiten 1 - 75) und H. Machud Yahya, Bernard T. Wahyu Wiryanta (in "Khasiat dan manfaat buah mehra si emas merah dari papua", Februar 2005, PT AgroMedia Pustaka, Jakarta, Seiten 1 - 84) traditionelle Arten zur Gewinnung von öligen Präparaten aus dem roten Fruchtfleisch der Pandanus-Frucht. Diese Präparate werden nicht durch Extraktion gewonnen, sondern durch Verkochung der Frucht mit Wasser und anschließendem Abschöpfen des sich absetzenden Öls. Insgesamt umfassen diese traditionellen Verfahren ein Erhitzen bei 100°C über 6 bis 8 Stunden. Die Öl-Ausbeute nach dem Stand der Technik ist sehr niedrig und beträgt ca. 2-3 %. Zudem ist bisher noch nicht berichtet worden, dass auch die Kerne und dass innere helle Fruchtfleisch extrahiert werden können, da man Kerne und helles Fruchtfleisch für giftig hält.

Die vorliegende Erfindung stellt nun Extrakte und deren Verwendung bereit, welche bisher noch nicht offenbart worden sind und als unverträglich und giftig gehalten wurden. Kerne und helles Fruchtfleisch können zerkleinert und danach extrahiert werden.

Für die Extraktion werden bevorzugt Hexan, Heptan, Cyclohexan, Chloroform, Methylenchlorid, Essigester, Diethylether, Petrolether, tert-Butylmethylether, THF, Methanol, Ethanol, Propanol, iso-Propanol, Butanol, iso-Butanol, sec-Butanol, Aceton und Mischungen dieser Lösungsmittel verwendet. Nach der Extraktion wird das Lösungsmittel vorzugsweise wieder entfernt und recyclisiert, um für weitere Extraktionen erneut verwendet zu werden. Das aufkonzentrierte Öl enthält die pharmakologisch wirksamen Bestandteile des extrahierten Fruchtfleisches.
Ein weiteres bevorzugtes Extraktionsverfahren ist eine Kombination aus Kaltpressung und Extraktion der Kerne, indem zuerst das rote Fruchtfleisch samt Kerne bei Temperaturen unterhalb von vorzugsweise 40°C gepresst werden und danach das verbliebene Fruchtfleisch mit der Kernen zermahlen wird. Vor dem Zermahlen kann Fruchtfleisch und Kerne auch getrocknet werden. Die zerriebenen Kerne samt verbliebenem zerriebenem Fruchtfleisch werden danach analog des Verfahrens zur Extraktion der Kerne weiterbehandelt.

Ein weiteres Verfahren der vorliegenden Erfindung betrifft die Herstellung eines hydrophilen Extraktes aus den abgetrennten Kernen der Frucht der Spezies Pandanus conoideus durch Zerkleinerung der Kerne bei Temperaturen unterhalb von vorzugsweise 40°C und Extraktion der zerkleinerten Kerne mit einem hydrophilen Lösungsmittel bei Temperaturen unterhalb von vorzugsweise 40°C. Es gilt, dass die Extraktionen bei Temperaturen zwischen 4°C und 100°C durchgeführt werden können, vorzugsweise jedoch Temperaturen von 60°C und insbesondere bevorzugt Temperaturen von 40°C nicht überschritten werden sollten.

Als hydrophiles Lösungsmittel werden vorzugsweise Wasser, Aceton, Tetrahydrofuran (THF), Methanol, Ethanol, Propanol, iso-Propanol, Butanol oder Mischungen dieser Lösungsmittel eingesetzt, wobei Wasser, Methanol und Ethanol oder Mischungen aus Wasser, Methanol und Ethanol bevorzugt sind. Nach eventuell mehrfacher Extraktion und Vereinigung der hydrophilen Extrakte, wird das Lösungsmittel wieder entfernt und erneut verwendet. Diese Schritte sollten vorzugsweise bei Temperaturen unterhalb von 40°C durchgeführt werden. Der aufkonzentrierte Extrakt enthält die pharmakologisch aktiven Bestandteile aus den Kernen dieser Frucht.

Ein weiteres Verfahren der vorliegenden Erfindung betrifft die Herstellung eines hydrophilen Extraktes aus dem weißen Fruchtfleisch der Früchte der Spezies Pandanus conoideus durch eine direkte Verpressung des abgetrennten weißen Fruchtfleisches mit oder ohne Zusätze von Antioxydantien bei Temperaturen vorzugsweise unterhalb von 40°C oder durch Trocknung und Zerkleinerung des abgetrennten weißen Fruchtfleisches bei Temperaturen unterhalb von vorzugsweise 40°C und anschließender Extraktion des getrockneten zerkleinerten weißen Fruchtfleisches mittels hydrophiler Lösungsmittel.

Als hydrophiles Lösungsmittel werden vorzugsweise Wasser, Aceton, Tetrahydrofuran (THF), Methanol, Ethanol, Propanol, iso-Propanol, Butanol oder Mischungen dieser Lösungsmittel eingesetzt, wobei Wasser, Methanol und Ethanol oder Mischungen aus Wasser, Methanol und Ethanol bevorzugt sind. Nach eventuell mehrfacher Extraktion des weißen bzw. hellen Fruchtfleisches und Vereinigung der hydrophilen Extrakte, wird das Lösungsmittel wieder entfernt und kann erneut verwendet werden. Alle Extraktionsschritte sollten vorzugsweise bei Temperaturen unterhalb von 40°C durchgeführt werden. Der aufkonzentrierte Extrakt enthält die pharmakologisch aktiven Bestandteile aus dem weißen Fruchtfleisch der Pandanus conoideus.

Die Frucht des Pandanus conoideus besteht aus einem inneren hellen Fruchtanteil und einem roten, kernhaltigen und ölhaltigen äußeren Fruchtanteil. Die vorliegende Erfindung offenbart 5 verschiedene Extrakte mit unterschiedlichen pharmakologischen Aktivitäten, welche aus der Frucht des Pandanus conoideus gewonnen werden können.

Zur Gewinnung der biologisch aktiven Extrakte werden die Früchte gereinigt und die rote, äußere Fruchtschicht von dem weißen/hellen, inneren Fruchtanteil abgetrennt. Die verschiedenen erfindungsgemäßen Extrakte könnten auf verschiedene Arten erhalten werden, wie beispielsweise in den Beispielen 1 - 7 offenbart ist.

Sämtliche Arbeitsschritte werden bevorzugt bei Temperaturen von 17 - 40°C und insbesonder bevorzugt bei 19°C - 38°C durchgeführt.

Ein vereinfachtes Verfahren zur Gewinnung eines Extraktes aus dem roten Fruchtfleisch besteht darin, dass zuerst das rote Fruchtfleisch von den Kernen zu trennen, danach zu zerkleinern und mit einem organischen Lösungsmittel mehrmals zu extrahieren. Verwendet man für die Extraktion Ethanol, so kann dieser alkoholische Extrakt direkt als Medizin eingesetzt werden. Dieses Verfahren ist insbesondere für die Verwendung von physiologisch verträglichen Lösungsmitteln geeignet und vorzugsweise für Alkohole wie Ethanol, Isopropanal und insbesondere für Ethanol.

Ein vereinfachtes Verfahren zur Gewinnung eines alkoholischen Extraktes aus dem hellen Fruchtfleisch besteht darin, das helle weiße innere Fruchtfleisch zu trocknen, bevorzugt an der Luft, danach zu zerkleinern und mit einem hydrophilen Lösungsmittel mehrmals zu extrahieren. Verwendet man für die Extraktion Ethanol oder Ethanol-Wasser-Gemische, so kann dieser alkoholische Extrakt direkt als Medizin eingesetzt werden.

Eine weitere Möglichkeit besteht daran, das innere helle Fruchtfleisch kalt zu pressen (bevorzugt t<40°C). Der erhaltene wäßrige Extrakt kann optional noch mit Geschmacksstoffen, Farbstoffen und/oder Konservierungsmittel versetzt werden und kann in der vorliegenden Form als Arzneistoff verwendet werden. Natürlich kann der wäßrige Extrakt auch als Saft verabreicht oder getrocknet werden und der so erhaltene Rückstand z.B. als Kapsel oder Tablette formuliert werden.

Erfolgt die Extraktion der abgetrennten und zerkleinerten Kerne mittels Ethanol, so kann auch dieser ethanolische Extrakt unmittelbar als Medizin eingesetzt werden.

Somit betrifft die vorliegende Erfindung auch insbesondere ethanolische Extrakte, welche unmittelbar bei schonenden Bedingungen (t<40°C) aus dem roten, aus dem hellen bzw. weißen Fruchtfleisch sowie die aus den Kernen gewonnen wurden. Die gewonnene alkoholische Lösung kann eingeengt werden und kann zur Zubereitung einer ethanolischen Lösung oder eines Feststoffs nach Trocknung dienen, wobei der Feststoff als aktive Komponente üblichen Formulierungen zugesetzt werden kann.

Die vorliegende Erfindung ist somit auf ein Extraktionsverfahren für Pandanus conoideus gerichtet, welches die folgenden Schritte umfasst:
1) Trennung der Frucht in helles, weißes inneres Fruchtfleisch sowie rotes Fruchtfleisch und Kerne,
2) Kaltpressung des roten Fruchtfleisches, um die Ölfraktion zu gewinnen, und
3) Extraktion des Rückstandes des roten Fruchtfleisches mit Alkohol (z.B. Methanol, Ethanol, Propanol, iso-Propanol, Butanol) bei Temperaturen unterhalb von 40°C; oder
4) Extraktion des Öls direkt aus dem roten Fruchtfleisch mit Alkohol oder anderen organischen Lösungsmitteln bei Temperaturen unterhalb von 40°C,
5) Extraktion der Kerne mit hydrophilen Lösungsmitteln,
6) Kaltpressen des weißen hellen Fruchtfleisches, um Saft zu gewinnen, oder
7) optional Extraktion des weißen hellen Fruchtfleisches mit hydrophilen Lösungsmitteln, und/oder
8) Trocknung des weißen hellen Fruchtfleisches, und
9) anschließende Extraktion des weißen hellen Fruchtfleisches mit hydrophilen Lösungsmitteln.

Ähnliche Extraktionsverfahren, welche unter den Schutzumfang der vorliegenden Erfindung fallen, können wie folgt beschrieben werden und umfassen zumindest einige der folgenden Schritte:
a) Trennung der Frucht in helles weißes inneres Fruchtfleisch sowie rotes Fruchtfleisch und Kerne,
b) Kaltpressung des roten Fruchtfleisches, und
c) Zerkleinerung und/oder Homogenisierung des roten Fruchtfleisches, und
d) Extraktion des Saftes aus dem roten Fruchtfleisch sowie des zerkleinerten oder homogenisierten roten Fruchtfleisches mit Alkoholen oder anderen organischen lipophilen Lösungsmitteln bei Temperaturen unterhalb von 40°C. und
e) Sammlung und Einengung der lipophilen Extrakte, oder
f) Zerkleinerung der Kerne und
g) Extraktion der zerkleinerten Kerne mit hydrophilen Lösungsmitteln, und
h) Sammlung und Einengung der hydrophilen Extrakte, oder
i) Kaltpressen des weißen hellen inneren Fruchtfleisches, oder Zerkleinerung und/oder Homogenisierung des getrockneten weißen hellen Fruchtfleisches, und
j) Extraktion des zerkleinerten oder homogenisierten getrockneten weißen hellen Fruchtfleisches mit hydrophilen Lösungsmitteln, und
k) Sammlung und Einengung der hydrophilen Extrakte.

Auch die lipophile Fraktion (das Öl) aus Pandanus conoideus kann direkt, ohne weitere Verarbeitung gegen unterschiedliche Erkrankungen eingesetzt werden (Dosierung 1-3 Eßlöffel pro Tag) oder direkt in Softgelkapseln verarbeitet werden.

Ein Extrakt oder mehrere Extrakte aus der Pandanus conoideus wird/werden vorzugsweise zur Herstellung einer pharmazeutischen Zusammensetzung verwendet, welche den Extrakt oder die Extrakte aus Pandanus conoideus zusammen mit mindestens einem pharmakologisch verträglichen Hilfsstoff, Trägerstoff und/oder Lösungsmittel enthält.

Die vorliegende Erfindung offenbart Verfahren, nach denen aus der Frucht der Pflanze Pandanus conoideus 5 verschiedene Extrakte gewonnen werden können. Diese 5 Extrakte zeigen alle pharmakologische Aktivität, sind jedoch gegen verschiedene Erkrankungen unterschiedlich stark aktiv. Somit ist es erfindungsgemäß bevorzugt, zwei oder auch mehrere erfindungsgemäße Extrakte miteinander zu mischen oder zu kombinieren. Demnach ist es bevorzugt, einen Extrakt aus dem roten Fruchtfleisch mit einem Extrakt aus den Kernen und/oder mit einem Extrakt aus dem hellen weißen Fruchtfleisch zu mischen.

Genauso kann auch ein Extrakt aus den Kernen mit einem Extrakt aus dem hellen weißen Fruchtfleisch und/oder mit dem Saft nach Kaltpressung des hellen weißen Fruchtfleisches und/oder mit einem Extrakt aus dem roten Fruchtfleisch und/oder mit einem Extrakt aus dem nach Kaltpressung des roten Fruchtfleisches erhaltenen Saft bzw. den Bestandteilen des Saftes nach dessen Trocknung hergestellt werden.

Ein Extrakt aus dem weißen getrockneten hellen Fruchtfleisch und/oder aus dem durch- Kaltpressung des weißen hellen Fruchtfleisches gewonnenen Saft kann kombiniert werden mit einem Extrakt aus den Kernen und/oder einem Extrakt aus dem roten Fruchtfleisch, und/oder mit einem aus dem Kaltpressung des roten Fruchtfleisches erhaltenen Öl bzw. den Bestandteilen des Öles nach dessen Trocknung.

Durch derartige Kombinationen kann das Wirkspektrum der Kombination von Extrakten bzw. der diese Kombination von Extrakten enthaltenden pharmazeutischen Formulierung verbreitert und die Aktivität auch teilweise gesteigert werden.
Die Herstellung der Extrakte aus dem roten Fruchtfleisch oder aus den abgetrennten Kernen oder aus dem hellen, inneren Anteil der Frucht kann auch durch Extraktion mit überkritischen Fluiden (Kohlendioxid, Propan, Butan), vorzugsweise mit Kohlendioxid, mit oder ohne zusätzlichem Einsatz von Schleppmitteln (Methanol, Ethanol) durchgeführt werden. Mittels der vorgenannten Fluide lassen sich bevorzugt lipophile Extrakte erhalten, wobei durch gleichzeitige Verwendung von polaren Schleppmitteln auch weniger lipophile und eher polare bis hydrophile Extrakte gewonnen werden können. Somit werden bei der Extraktion des roten Fruchtfleisches bevorzugt keine polaren Schleppmittel eingesetzt, wohingegen die Verwendung von polaren Schleppmitteln bei der Extraktion der Kerne und des inneren hellen Fruchtfleisches bevorzugt ist.

Die durch Extraktion mit überkritischen Fluiden (Kohlendioxid, Propan, Butan) vorzugsweise mit Kohlendioxid gewonnenen Extrakte können natürlich einzeln oder in Kombination miteinander als auch in Kombination mit den nicht durch überkritische Fluide erhaltenen Extrakte eingesetzt werden.

Unter dem Begriff Extrakt soll sowohl die flüssige Phase nach einer einmaligen Extraktion als auch die kombinierten flüssigen Phasen von mehreren Extraktionen verstanden werden. Zudem soll der Begriff Extrakt auch die eingeengte flüssige Phase umfassen, bei welcher das Lösungsmittel teilweise, weitgehend oder vollständig entfernt worden ist, so dass Extrakt auch ein fester Rückstand bedeuten kann, der nach Entfernung des Lösungsmittels vorzugsweise unter vermindertem Druck und nach Trocknung der festen Bestandteile erhalten worden ist.

Die erfindungsgemäßen Extrakte können alleine oder in Kombination mit anderen erfindungsgemäßen Extrakten und/oder in Kombination mit weiteren Wirkstoffen, Vitaminen, Spurenelementen, Mineralien so wie sie gewonnen wurden oder in Form pharmazeutischer Formulierungen und Zusammensetzungen gegen diverse hierin beschriebene Erkrankungen eingesetzt werden.

Die pharmazeutische Zusammensetzung enthaltend einen Extrakt oder mehrere Extrakte aus Pandanus conoideus kann mit den üblichen festen oder flüssigen Trägerstoffen oder Verdünnungsmitteln und den üblicherweise verwendeten pharmazeutischen Hilfsstoffen entsprechend der gewünschten Applikationsart mit einer geeigneten Dosierung in bekannter Weise hergestellt werden. Solche Darreichungsformen sind beispielsweise Tabletten, Minitabletten, Mikrotabletten, Filmtabletten, Schichttabletten, Dragees, Kapseln, Mikrokapseln, Mikropellets und Pellets, Pillen, Granulaten, Pulver, Puder, Lösungen Tropfen, Säfte, Suspensionen, Suppositorien, Klysma, Emulsionen, Dispersionen, Creme, Gelen, Salben, Sirup oder Depotformen oder Inhalätionslösungen bzw. Inhalationspulver. Zudem umfassen die erfindungsgemäßen pharmazeutischen Zusammensetzungen Formulierungen wie Schichttabletten zur kontrollierten und/oder kontinuierlichen Freisetzung des oder der Wirkstoffe sowie Mikroverkapselungen als spezielle Darreichungsformen.

Derartige Zubereitungen sind unter anderem für die Inhalation oder die intravenöse, intraperitoneale, intramuskuläre, subkutane, orale, rektale, transdermale, topikale, intradermale, intragastrale, intrakutane, intravaginale, intranasale, intrabuccale, perkutane oder sublinguale Verabreichung geeignet.

Besonders vorteilhafte Darreichungsformen sind die orale, rektale und topische Applikation, die Injektion, die Infusionen als auch die Inhalation.

Entsprechende Tabletten können beispielsweise durch Mischen der/des erfindurigsgemäß einsetzbaren Extrakte(s) mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Hydroxypropylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talkum, Titandioxid oder Zucker hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäß hergestellten Extrakt können zusätzlich geschmacksverbessernde Mittel wie Saccharin, Cyclamat oder Zucker sowie Aromastoffe wie Vanillin oder Orangenextrakt enthalten. Sie können außerdem Suspendierhilfsstoffe wie Natriumcarboxymethylcellulose oder Konservierungsmittel wie p-Hydroxybenzoate enthalten. Den Extrakt enthaltende Kapseln können beispielsweise hergestellt werden, indem man den Wirkstoff mit einem inerten Träger wie Milchzucker oder Sorbit mischt und in Gelatinekapseln einkapselt.

Die lipophile Fraktion (ölige Phase) kann auch direkt in Weichgelatin-Kapseln verarbeitet werden.

Geeignete Suppositorien lassen sich beispielsweise durch Vermischen mit dafür vorgesehenen Trägermitteln wie Neutralfetten oder Polyethylenglykol bzw. Derivaten davon herstellen.

Die lipophile Fraktion und/oder die Extrakte können auch als Klistier (rektale lnstillation) verabreicht werden.

Bestimmte Formulierungen sind unter anderem für die Inhalation oder die intravenöse, intraperitoneale, intramuskuläre, subkutane, orale, rektale, transdermale, topikale, intradermale, intragastrale, intrakutane, intravaginale, intranasale, intrabuccale, perkutane oder sublinguale Verabreichung geeignet.

Als pharmakologisch verträgliche Träger können beispielsweise Lactose, Stärke, Sorbitol, Sucrose, Cellulose, Magnesiumstearat, Dicalciumphosphate, Calciumsulfate, Talk, Mannitol, Ethylalkohol und dergleichen eingesetzt werden. Puder als auch Tabletten können zu 5 bis 95% aus einem derartigen Träger bestehen.

Als Bindemittel können zudem Stärke, Gelatine, natürliche Zucker, natürliche als auch synthetische Gummis wie beispielsweise Akaziengummi oder Guar-Gummi, Natriumalginat, Carboxymethyl-Cellulose, Polyethylenglycol und Wachse eingesetzt werden. Als Gleitmittel können Borsäure, Natriumbenzoat, Natriumacetat, Natriumchlorid, und dergleichen dienen.

Ferner können den pharmazeutischen Zusammensetzungen noch Sprengmittel, Farbstoffe, Geschmacksstoffe und/oder Bindemittel zugesetzt werden.

Flüssige Formulierungen umfassen Lösungen, Suspensionen, Sprays und Emulsionen. Beispielsweise Injektionslösungen auf Wasserbasis oder Wasser-Propylenglycol-Basis für parenterale Injektionen.

Für die Zubereitung von Suppositorien werden bevorzugt niedrigschmelzende Wachse, Fettsäureester und Glyceride eingesetzt.

Kapseln werden beispielsweise aus Methylcellulose, Polyvinylalkohole oder denaturierte Gelatine oder Stärke hergestellt.

Als Sprengmittel können Stärke, Natrium-Carboxymethylstärke, natürliche und synthetische Gummis wie beispielsweise Johannisbrotkernmehl, Karaya, Guar, Tragant und Agar sowie Cellulosederivate wie Methylcellulose, Natrium-Carboxymethylcellulose, microkristalline Cellulose sowie Alginate, Tonerden und Bentonite verwendet werden. Diese Bestandteile können in Mengen von 2 bis 30 Gew.-% eingesetzt werden. ,

Als Bindemittel können Zucker, Stärke aus Korn, Reis oder Kartoffeln, natürliche Gummis wie Akaziengummi, Gelatine, Tragant, Alginsäure, Natriumalginat, Ammonium-Calcium-Alginat, Methylcellulose, Natrium-Carboxymethylcellulose, Hydroxypropyl-methylcellulose, Polyvinylpyrrolidon sowie anorganische Verbindungen wie Magnesium-Aluminum-Silicate zugesetzt werden. Die Bindemittel könne in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin eingesetzt werden. Derartige Gleitmittel können in Mengen von 0,05 bis 15 Gew.-% verwendet werden.

Die erfindungsgemäße pharmazeutische Formulierung wird insbesondere in der Krebstherapie eingesetzt. Es ist möglich einer pharmazeutischen Formulierung ein Vitamin und/oder mindestens einen antiproliferativen, antiangiogenen, antiinflammatorischen, antiphlogistischen, cytostatischen und/oder cytotoxischen Wirkstoff zuzusetzen.

Als Vitamine können Vitamin A, Vitamin C (Ascorbinsäure), Vitamin D, Vitamin H, Vitamin K, Vitamin E, VitaminB1, VitaminB2, VitaminB3, VitaminB5, VitaminB6, VitaminB12, Thiamin, Riboflavin, Niacin, Pyridoxin und Folsäure verwendet werden.

Als antiproliferative, antiangiogene, cytostatische und/oder cytotoxische Wirkstoffe können der pharmazeutischen Zusammensetzung Chlorethamin, Cyclophosphamid, Trofosfamide, Ifosfamid, Melphalan, Chlorambucil, Busulfan, Thiotepa, Carmustin, Lomustin, Dacarbazin, Procarbazin, Temozolomid, Treosulfan, Estramustin, Nimustin, Daunorubicin als auch liposomales Daunorubicin, Doxorubicin, Adriamycin als auch liposomales Adriamycin, Dactinomycin, Mitomycin C, Bleomycin, Epirubicin (4-Epi-Adriamycin), Idarubicin, Dactinomycin, Mitoxantron, Mitomycin-C, Plicamycin, Amsacrin, Actinomycin D, Methotrexat, 5-Fluorouracil, 6-Thioguanin, 6-Mercaptopurin, Fludarabin, Cladribin, Pentostatin, Gemcitabin, Cytarabin, Azathioprin, Raltitrexed, Capecitabin, Cytosinarabinosid, Tioguanin, Mercaptopurin, Vincristin, Vinblastin, Vindesin, Etoposid, Teniposid, Cisplatin, Carboplatin, Oxaliplatin, Vinca-Alkaloide, Venorelbin, , Camptothecin, Irinotecan, Paclitaxel, Docetaxel, Hydroxycarbamide (Hydroxyurea), Imatinib, Miltefosin, Amsacrin, Topotecan (Topoisomerase-I-Inhibitor), , Bexaroten, Tretinoin, Asparaginase, Trastuzumab, Alemtuzumab, Rituximab, Glucocorticoide (Prednison), Oestrogene (Fosfestrol, Estramustin), LHRH (Buserelin, Goserelin, Leuprorelin, Triptorelin), Flutamid, Cyproteronacetat, Tamoxifen, Toremifen, Aminoglutethimid, Formestan, Exemestan, Letrozol, Anastrozol, Interleukin-2, Interferon-α, Erythropoietin, G-CSF, , Efitinib, Ibritumomab, Levamisol und/oder Retinoide zugesetzt werden.

Die erfindungsgemäßen pharmazeutischen Formulierungen oder die Extrakte insbesondere die ethanolischen Extrakte werden vorzugsweise zur Prophylaxe und Behandlung von Bauchspeicheldrüsenkrebs, und Lungenkrebs, eingesetzt.

### Figurenbeschreibung:

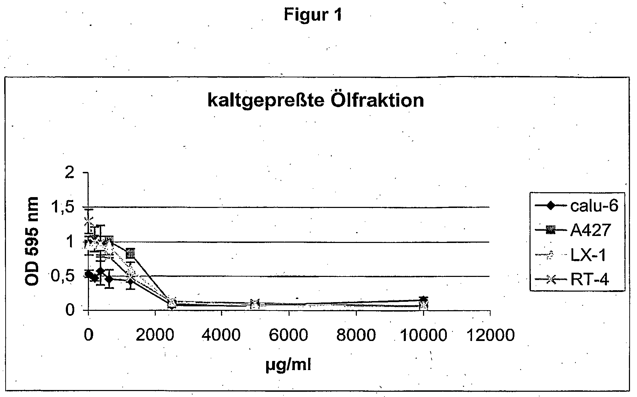

Figur 1 zeigt die antitumorale Wirkung einer aus der Frucht der Pflanze Pandanus conoideus gewonnenen kaltgepreßten Ölfraktion (Extrakt 1) auf die Zelllinien von Lungenkrebszellen LX-1, Calu-6, A-427 und Blasenkrebszellen RT-4.

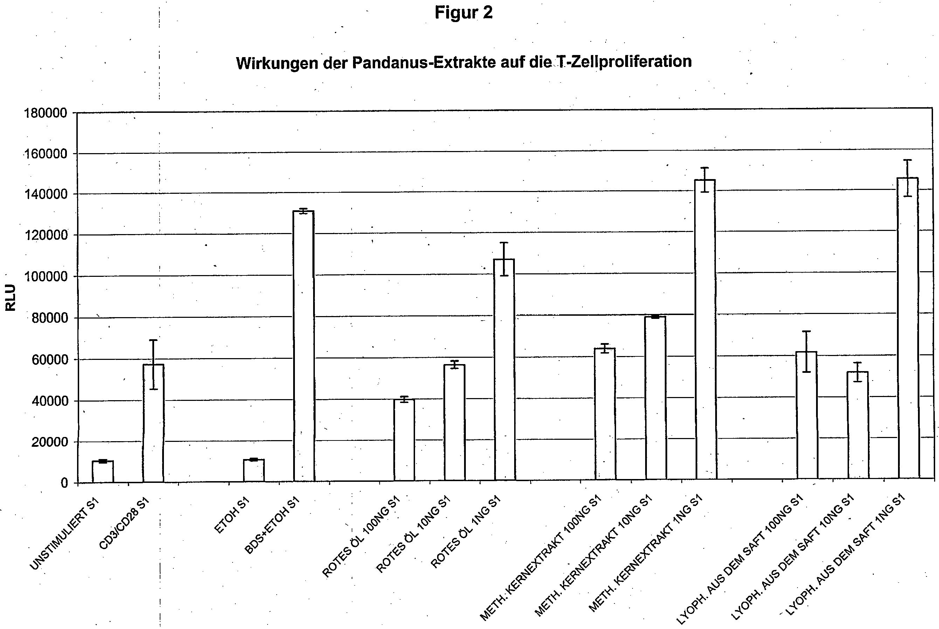

Figur 2 zeigt den Einfluß verschiedener Pandanus-Extrakte auf die T-Zell-proliferation. Hierbei werden T-Zellen aus dem Blut von gesunden Spendern isoliert und mit vesikel/bead-gekoppelten Antikörpern gegen CD3 und CD28 stimuliert. Die Kulturen werden mit oder ohne die zu testenden Extrakte (Rotes Öl = Extrakt 1, methanolischer Kernextrakt = Extrakt 4, Lyophilisat aus dem Saft = Extrakt 5) angesetzt und dadurch der Effekt der Extrakte auf die Proliferationsrate bestimmt. Die T-Zell-proliferation wird über einen ATP-abhängigen Lumineszenzassay nach 72h Inkubation gemessen (RLU = relative light unit; CD3/CD28 = zeigt die Wirkung von CD3/CD28 auf die Proliferation; ETOH = zeigt die Wirkung von Ethanol alleine; BDS+ETOH = Stimulation mit bead-gekoppelten Antikörpern gegen CD3 und CD28 plus Ethanol als Hilfsmittel).

### Beispiel 1:

### Gewinnung von kattoepreßtem Öl ()

Die rote, kernhaltige Fruchtschicht wird vorzugsweise mit Zusatz von Wasser in einem Mixer gerührt um das rote Fruchtfleisch von den Kernen zu trennen. Das gewonnene, rote Fruchtfleischbrei werden gepresst. Nach Zentrifugation und Filtration gewinnt man ein klares, rotes, dickflüssiges Öl (kalt gepresstes Virgin-Öl; Extrakt 1). Die Kerne werden mit Wasser gewaschen und bei einer Temperatur von höchsten 40°C im Trockenschrank getrocknet und für weitere Verarbeitung aufbewahrt.

### Beispiel 2:

### Gewinnung des Öls durch eine Extraktion mit organischen Lösungsmitteln

Das wie oben gewonnene, rote Fruchtfleischbrei wird mit einem lipophilen Lösungsmittel, z.B. Hexan, Pentan, Cyclohexan, Petrolether, Heptan, tert-Butylmethylether extrahiert:
Nach Trocknung (Wasserentzug), Zentrifugation und Filtration erhält man das rote, dickflüssige Öl.

### Beispiel 3:

### Gewinnung der Alkoholextrakte

Das wie oben gewonnene, rote Fruchtfleischbrei wird mit einem polaren Lösungsmittel wie beispielsweise einem Alkohol, Ether, Aceton, THF, Essigsäureethylester und bevorzugt Methanol oder Ethanol extrahiert.
Nach Abdampfen des Lösungsmittel erhält man den alkoholischen Extrakt.

### Beispiel 4:

### Gewinnung der Alkoholextrakte aus den Kernen

Die wie oben beschrieben gewonnenen Kerne werden zerkleinert und mit 50%igem Methanol-Wasser extrahiert. Nach Abdampfen des Lösungsmittels erhält man der Kernextrakt.

### Beispiel 5:

### Gewinnung des wässrigen Saftes aus der weißen/hellen, innere Fruchtschicht

Aus der weißen Schicht, d.h. dem inneren Fruchtfleischanteil wird durch direkte Verpressung eine wässrige Phase gewonnen. Stabilisatoren wie z.B. Ascorbinsäure oder andere Antioxydantien kann dem Saft zugefügt werden um Oxidation durch Luftsauerstoff zu verhindern. Der Saft kann direkt eingesetzt werden oder getrocknet für spätere Anwendung.

### Beispiel 6:

### Gewinnung der Alkoholextrakte aus dem inneren, hellen Fruchtanteil

Der weiße, helle Fruchtanteil wird in Scheiben von ca. 4x4x2 cm geschnitten und im Trockenschrank getrocknet. Die Stücke werden zerkleinert und mit 50% Methanol-Wasser oder 50% Ethanol-Wasser extrahiert.

### Beispiel 7:

### Extraktgewinnung aus dem roten Fruchtfleisch mit überkritischem Kohtendioxid (Tk = 31°C, Pk = 73,9 bar)

Im Extraktionsbehälter wird das rote Fruchtbrei mit dem flüssigen Kohlendioxid versetzt (40°C, 90 bar, 8 kg CO₂/h über 4 h). Das mit Pflanzeninhaltsstoffen beladene Lösungsmittel wird mit Hilfe einer Pumpe in den Abscheidebehälter gepumpt. Auf dem Weg dorthin wird der Druck mittels eines Drosselventils auf unterkritische Bedingungen entspannt. Die Löslichkeit nimmt ab, und die gelösten lipophilen Stoffe fallen aus. Im Abscheidebehälter werden die lipohilen Stoffe (Ölfraktion) vom Gas getrennt. Das Gas wird zum Fluid rekomprimiert und wieder verwendet.
Durch Zugabe verschiedener Konzentrationen von Schleppmitteln (z.B. Methanol oder Ethanol) können auch mittelpolare und auch polare Substanzen schonend extrahiert werden.

### Beispiel 8: Zubereitung der Extrakte

Nach dem gründlichen Waschen der Frucht mit Wasser wird die äußere rote Schicht von der inneren weißen Schicht getrennt. Aus der roten Schicht wird durch Schütteln/Reiben der Fruchtfleisch-Anteil von den Kernen getrennt.

Der roten Fruchtfleisch-Anteil wird homogenisiert, gepresst und zentrifugiert. Nach der Zentrifugation wird die Ölphase abgetrennt (Extrakt 1). Der Rückstand wird zuerst mit n-Hexan (Extrakt 2) und danach mit Methanol (Extrakt 3) extrahiert.

Die Kerne werden zerkleinert und mit 50% Methanol extrahiert (Extrakt 4).

Aus der weißen Schicht wird durch die direkte Verpressung eine wässrige Phase (Extrakt 5) gewonnen. Der Rückstand nach der Verpressung wird getrocknet und mit 50% Methanol extrahiert (Extrakt 6).

Alle Arbeitsvorgänge werden unter 40° C durchgeführt.

### Ergebnisse:

Alle Fraktionen, mit Ausnahme Extrakt 6 die nach dieser Methode hergestellt wurden, zeigten antitumorale Aktivitäten (Tab. 1).
Tabelle 2 zeigt die IC-50 Werte der Ölfraktion (Extrakt 1) gegen verschiedene Krebszelllinien.
Tabelle 3 zeigt die IC-50-Werte des lyophilisierten Saftes (Extrakt 5) gegen verschiedene Krebszelllinien.

### Beispiel 9:

### Antitumorale Aktivität der verschiedenen Extrakte von Pandanus conoideus

### Zellkultur

Die Zellen wurden in 50 ml Zellkulturflaschen (Greiner) in einem RPMI 1640 Medium (Sigma, München), dem je 50 ml Zellkultur 10% fötales Kälberserum (Sigma, München) zugesetzt wurden, bei 37°C in feuchter, 5%iger CO₂ Atmosphäre aufbewahrt und 1 bis 2 mal wöchentlich mit frischem Medium versehen.

### Protokoll für den Cytotoxizitätstest

Monoschichten wurden durch Dispensieren von 2 x 10⁶ Tumorzellen in 96-Lochplatten (Greiner) zubereitet. Die Testsubstanzen wurden entweder in Wasser oder Aceton mit einer Konzentration bis zu 10 mg/ml gelöst.

Die Aktivitäten wurden semiquantitativ und quantitativ bestimmt. Bei den semiquantitativen Bestimmungen wurden Verdünnungen angewendet um eine maximale Zelltod (100% Zelltod) zu erreichen (Verdünnung 1:64 = starke Aktivität/ +++; 1:32 = mittelstarke Aktivität/ ++; 1:16 = schwache Aktivität/ +; keine Aktivität/ 0). Bei den quantitativen Bedingungen wurden die IC-50-Werte bestimmt.

Alle Assays wurden dreimal ausgeführt. Nach Ablauf von drei Tagen gerechnet vom Beginn der Behandlung wurden die Zellen mittels 3%iger Formaldehydlösung fixiert und das Medium dekantiert. Die Zellen wurden mit Kristallviolett gefärbt. Die optische Dichte bei 595 nm (OD595) wurde mittels eines Multiscan Ascent ELISA Readers bestimmt.

Folgende Zelllinien wurden verwendet: Calu-6 Lungenkrebszellen, A 427 Lungenkrebszellen, LX-1 Lungenkrebszellen, RT-4 Blasenkrebszellen, Capan-2 Bauchspeicheldrüsenkrebszellen, MCF-7 Brustkrebszellen.

### Ergebnisse:

**Tabelle 1: Aktivitäten der verschiedenen Extrakte (Extrakt 1 - 6) auf diverse Krebszelllinien:**

| Zelllinien / Extrakte | **1** | **2** | **3** | **4** | **5** | **6** |
|---|---|---|---|---|---|---|
| Calu-6 | +++ | +++ | ++ | +++ | +++ | 0 |
| A 427 | ++ | NB | NB | NB | NB | NB |
| LX-1 | ++ | NB | NB | NB | NB | NB |
| RT-4 | +++ | NB | NB | NB | NB | NB |
| Capan-2 | ++ | ++ | +++ | NB | ++ | NB |
| MCF-7 | + | + | +++ | NB | + | NB |

Die Aktivitäten wurden semiquantitativ bestimmt. Es wurden Verdünnungen angewendet um eine maximale Zelltod (100% Zelltod) zu erreichen (Verdünnung 1:64 = starke Aktivität/ +++; 1:32 = mittelstarke Aktivität/ ++; 1:16 = schwache Aktivität/ +; keine Aktivität/ 0, nicht bestimmt = NB).

### Extrakte:

### Rotes Fruchtfleisch

Extrakt 1 = Ölfraktion.
Extrakt 2 = Hexanextrakf
Extrakt 3 = Methanolextrakt aus dem Rückstand

### Kerne

Extrakt 4 = Methanolischer Kernextrakt

### Helles inneres Fruchtfleisch

Extrakt 5 = Wässriger Saft
Extrakt 6 = Methanolextrakt aus dem Rückstand nach Saftgewinnung

**Tabelle 2 . IC-50 Werte (ug/ml) der Ölfraktion (Extrakt 1)**

| IC50 (µp/ml] | |
|---|---|
| Zelllinien | |
| Calu-6 | 1556,35 |
| A427 | 1779,26 |
| LX-1 | 1497,64 |
| RT-4 | 846,25 |

**Tabelle 3: IC₅₀ Werte (ug/ml) vom lyophilisierten Saft (Extrakt 5)**

| IC50 [µg/ml] | |
|---|---|
| Zelllinien | |
| Capan-2 | 967,88 |
| MCF-7 | 1036,04 |
| Calu-6 | 776,25 |

### Beispiel 10

### Einflüsse der Pandanus-Extrakte auf das Immunsystem

Um zu prüfen, ob Pandanus-Extrakte das Immunsystem zu beeinflussen vermögen, wurden die Wirkungen der Extrakte auf die stimulierte T-Zellproliferation untersucht. Hierbei wurden T-Zellen aus dem Blut von gesunden Spendern isoliert (durch untouched isolation via magnetic beads) und mit bead-gekoppetten Antikörpern gegen CD3 und CD28 stimuliert. Die Kulturen wurden mit oder ohne Extrakte in verschiedenen Konzentrationen angesetzt und die Proliferationsrate bestimmt. Die T-Zellproliferation wurde über Lumineszenzassay nach 72 h Inkubation gemessen.

Figur 2 zeigt eindeutig, dass sowohl das Öl (Extrakt 1) als auch der Kernextrakt (Extrakt 4) und auch der lyophilisierte Saft (Extrakt 5) in der Lage sind konzentrationsabhängig die T-Zellproliferation zu beeinflussen. Als Träger immunologischer Funktionen sind die T-Zellen von zentraler Bedeutung für die Immunabwehr. Die Ergebnisse zeigen, dass die Anwendung der Pandanus-Extrakte bei immunologischen Erkrankungen wie bei Morbus Crohn und Colitis ulcerosa und bei entzündlichen Erkrankungen wie bei Rheumatoide Erkrankungen durchaus erfolgversprechend und berechtigt ist.

## Patentansprüche

1. Verfahren zur Herstellung eines lipophilen Extraktes aus dem roten Fruchtfleisch der Früchte der Spezies Pandanus conoideus durch direkte Verpressung des abgetrennten roten Fruchtfleisches oder durch Extraktion des abgetrennten zerkleinerten roten Fruchtfleisches bei Temperaturen unterhalb von 40°C.

2. Verfahren nach Anspruch 1, wobei die direkte Verpressung des abgetrennten roten Fruchtfleisches ohne jegliche chemische Zusätze durchgeführt wird.

3. Verfahren zur Herstellung eines hydrophilen Extraktes aus den abgetrennten Kernen der Frucht der Spezies Pandanus conoideus durch Zerkleinerung der Kerne und Extraktion der zerkleinerten Kerne mit einem hydrophilen Lösungsmittel.

4. Verfahren nach Anspruch 3, wobei die Zerkleinerung der Kerne und/oder die Extraktion bei Temperaturen unterhalb von 40°C durchgeführt werden.

5. Verfahren zur Herstellung eines hydrophilen Extraktes aus dem weißen/hellen/inneren Fruchtfleisch der Früchte der Spezies Pandanus conoideus durch eine direkte Verpressung des abgetrennten weißen Fruchtfleisches, mit oder ohne Zusätze von Antioxidantien, oder durch Trocknung und Zerkleinerung des abgetrennten weißen Fruchtfleisches und anschließender Extraktion des getrockneten zerkleinerten weißen Fruchtfleisches mittels hydrophiler Lösungsmittel.

6. Verfahren nach Anspruch 5, wobei die Verpressung und die Extraktion ohne jegliche chemische Zusätze durchgeführt werden und/oder die Verpressung und die Zerkleinerung des Fruchtfleisches als auch die Extraktion bei Temperaturen unterhalb von 40°C durchgeführt werden.

7. Verfahren nach Anspruch 1, wobei für die Extraktion Wasser, Essigsäureethylester, Ethanol, Isopropanol und Mischungen davon verwendet werden.

8. Verfahren nach Anspruch 3, 4, 5 oder 6, wobei für die Extraktion Methanol, Ethanol, Propanol, Isopropanol, THF, Aceton, Wasser und Mischungen davon und vorzugsweise Methanol und/oder Ethanol verwendet werden.

9. Verfahren zur Herstellung eines Extraktes aus dem roten Fruchtfleisch und/oder den Kernen und/oder aus dem inneren, hellen Fruchtfleisch der Früchte der Spezies Pandanus conoideus mittels überkritischen Fluiden.

10. Extrakt erhältlich durch direkte Verpressung oder durch Extraktion nach dem Verfahren gemäß eines der Ansprüche 1-9.

11. Verwendung des Extraktes gemäß Anspruch 10 zur Herstellung einer pharmazeutischen Zusammensetzung zur Prophylaxe und/oder Behandlung von Tumoren oder Krebserkrankungen, wobei die Tumoren oder Krebserkrankungen, aus der Gruppe ausgewählt werden umfassend Bauchspeicheldrüsenkrebs, Blasenkrebs, Lungenkrebs und Brustkrebs.

12. Kombination von mindestens zwei Extrakten, welche jeweils nach einem Verfahren gemäß den Ansprüchen 1, 2, 3, 4, 5, 6, 7, 8 oder 9 erhalten werden.

13. Pharmazeutische Zusammensetzung enthaltend mindestens einen Extrakt aus Pandanus conoideus, welcher nach einem Verfahren gemäß einen der Ansprüche 1-9 erhalten wurde, zusammen mit mindestens einem pharmakologisch verträglichen Hilfsstoff, Trägerstoff, Lösungsmittel, mindestens einem Vitamin und/oder mindestens einem antiproliferativen, antiinflammatorischen, cytostatischen und/oder cytotoxischen Wirkstoff.

## Claims

1. Method for preparing a lipophilic extract of the red pulp from the fruit of the species Pandanus conoideus by direct pressing of the separated red pulp or by extraction of the separated, crushed red pulp at temperatures below 40 °C.

2. Method according to claim 1, wherein the direct pressing of the separated red pulp is carried out without any chemical additives.

3. Method for preparing a hydrophilic extract of the separated seeds from the fruit of the species Pandanus conoideus by crushing the seeds and extracting the crushed seeds with a hydrophilic solvent.

4. Method according to claim 3, wherein the crushing of the seeds and/or the extraction is carried out at temperatures below 40 °C.

5. Method for preparing a hydrophilic extract of the white/pale/inner pulp from the fruit of the species Pandanus conoideus by directly pressing the separated white pulp with or without addition of antioxidants, or by drying and crushing the separated white pulp and subsequently extracting the dried, crushed white pulp by means of hydrophilic solvents.

6. Method according to claim 5, wherein the pressing and the extraction are carried out without any chemical additives and/or the pressing and the crushing of the pulp as well as the extraction are carried out at temperatures below 40 °C.

7. Method according to claim 1, wherein for the extraction water, acetic ester, ethanol, iso-propanol and mixtures thereof are used,

8. Method according to claims 3, 4, 5 or 6, wherein for the extraction, methanol, ethanol, propanol, iso-propanol, THF, acetone, water and mixtures thereof and preferably methanol and/or ethanol are used.

9. Method for preparing an extract of the red pulp and/or the seeds and/or the inner white pulp from the fruit of the species Pandanus conoideus by means of supercritical fluids.

10. Extract which can be obtained by direct pressing or by extraction according to the method of one of the claims 1-9.

11. Use of the extract according to claim 10 for the preparation of a pharmaceutical composition for the prophylaxis and/or the treatment of tumors or cancers, wherein the tumors or cancers are selected from the group comprising, pancreatic cancer, bladder cancer, lung cancer and breast cancer.

12. Combination of at least two extracts which are respectively obtained according to a method according to the claims 1, 2, 3, 4, 5, 6, 7, 8 or 9.

13. Pharmaceutical composition containing at least one extract of Pandanus conoideus which was obtained in accordance with a method pursuant to any of the claims 1 - 9 with at least one pharmacologically acceptable auxiliary agent, carrier, solvent, at least one vitamin and/or at least one antiproliferative, anti-inflammatory, cytostatic and/or cytotoxic active agent.

## Revendications

1. Procédé pour la préparation d'un extrait lipophile à partir de la pulpe rouge du fruit de l'espèce Pandanus conoideus par compression directe de la pulpe rouge séparée ou par extraction de la pulpe rouge séparée et broyée à une température inférieure à 40°C.

2. Procédé selon la revendication 1, dans lequel la compression directe de la pulpe rouge séparée est effectuée sans aucun additif chimique.

3. Procédé pour la préparation d'un extrait hydrophile à partir des noyaux séparés du fruit de l'espèce Pandanus conoideus par broyage des noyaux et extraction des noyaux broyés avec un solvant hydrophile.

4. Procédé selon la revendication 3, dans lequel le broyage des noyaux et/ou l'extraction sont effectués à une température inférieure à 40°C.

5. Procédé pour la préparation d'un extrait hydrophile à partir de la pulpe blanche/claire/intérieure de l'espèce Pandanus conoideus par compression directe de la pulpe blanche séparée, avec ou sans ajout des antioxydants, ou par séchage et broyage de la pulpe blanche séparée et extraction ultérieure de la pulpe blanche séchée et broyée au moyen d'un solvant hydrophile.

6. Procédé selon la revendication 5, dans lequel la compression et l'extraction sont effectuées sans aucun additif chimique et/ou la compression et le broyage de la pulpe, ainsi que l'extraction sont effectués à une température inférieure à 40°C.

7. Procédé selon la revendication 1, dans lequel de l'eau, de l'acétate d'éthyle, de l'éthanol, de l'isopropanol et des mélanges de ceux-ci sont utilisés pour l'extraction,

8. Procédé selon la revendication 3, 4, 5 ou 6, dans lequel du méthanol, de l'éthanol, du propanol, de l'isopropanol, du tétrahydrofurane, de l'acétone, de l'eau et des mélanges de ceux-ci et préférablement du méthanol et/ou de l'éthanol sont utilisés pour l'extraction.

9. Procédé pour la préparation d'un extrait à partir de la pulpe rouge et/ou des noyaux et/ou de la pulpe blanche et intérieure du fruit de l'espèce Pandanus conoideus au moyen d'un fluide supercritique.

10. Extrait obtenu par compression directe ou extraction selon le procédé d'une des revendications 1 - 9.

11. Utilisation de l'extrait selon la revendication 10 pour la préparation d'une composition pharmaceutique pour la prophylaxie et/ou le traitement des tumeurs ou des cancers, où les tumeurs ou les cancers sont sélectionnées à partir du groupe comprenant le cancer du pancréas, le cancer de la vessie, le cancer du poumon et le cancer du sein.

12. Combinaison d'au moins deux extraits, ou chacun des extraits est obtenu par un procédé selon la revendication 1, 2, 3, 4, 5, 6, 7, 8 ou 9.

13. Composition pharmaceutique comprenant au moins un extrait de Pandanus conoideus obtenu par un procédé selon une des revendications 1 - 9 avec au moins un adjuvant, un véhicule, un solvant pharmaceutiquement acceptable, au moins une vitamine et/ou au moins un agent antiprolifératit, anti-inflammatoire, cytostatique et/ou cytotoxique.
